# EUROPÄISCHE PATENTANMELDUNG

(11) **EP 0 738 719 A1**
(43) Veröffentlichungstag der Anmeldung: **23.10.1996**
(21) Anmeldenummer: 96105690.0
(22) Anmeldetag: 11.04.1996
(51) Int. Cl.: C07D 251/54, C07D 403/04, C07D 401/04

(54) **Formaldehydfreie Vernetzer für Lackbindemittel**

(30) Priorität: 18.04.1995 DE 19514318
(71) Anmelder: Vianova Resins GmbH, 55252 Mainz-Kastel (DE)
(72) Erfinder: Wonner, Johann, Dr., 60386 Frankfurt am Main (DE); Scholl, Frank, 63543 Neuberg (DE); Sauer, Josef, Dr., 63755 Alzenau (DE); Wölfert, Günter, 60318 Frankfurt am Main (DE)

(57) **Zusammenfassung**

Die vorliegende Erfindung betrifft Verbindungen der allgemeinen Formel IV 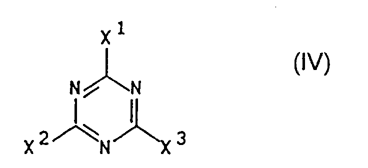 worin X¹, X² und X³ unabhängig voneinander für Amino, eine Gruppe der allgemeinen Formel V 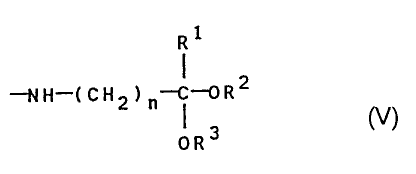 oder eine Gruppe der allgemeinen Formel VI 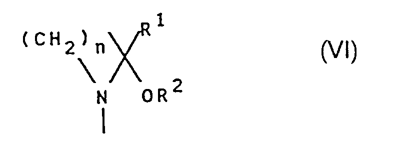 oder eine Gruppe der allgemeinen Formel VII 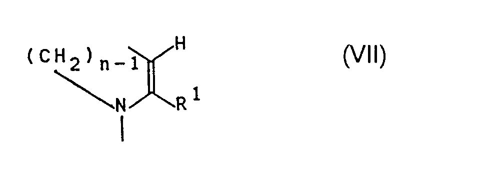 stehen; und R¹, R², R³ und n wie in Anspruch 1 angegeben definiert sind, und ihre Verwendung als Vernetzer für hydroxyl- oder aminogruppenhaltige Lackbindemittel.

## Beschreibung

Die vorliegende Erfindung betrifft formaldehydfreie Vernetzer auf Basis von acetal- bzw. ketalfunktionalisierten Melaminderivaten für hydroxyl- oder aminogruppenhaltige Lackbindemittel.

Der Einsatz von mit verschiedenen Alkoholen wie z.B.Methanol, n- oder iso-Butanol veretherten Melaminformaldehydharzen als Vernetzer von filmbildenden Copolymeren und/oder Cokondensaten ist schon lange bekannt (D. Plath, Kunststoffhandbuch Bd. 10 Duroplaste, Hanser Verlag München 1988, S. 994-996). Als alleinige Bindemittel sind veretherte Melaminharze jedoch ungeeignet, da die hohe Vernetzungsdichte der Melaminharze spröde Beschichtungen ergibt. Melaminharze werden aus diesem Grund ausschließlich in Kombination mit plastifizierenden Partnerharzen verarbeitet. Hierbei handelt es sich überwiegend um niedermolekulare Polymerisate oder Kondensate OH-gruppen-haltiger Monomere, die allein keine widerstandsfähigen, gut haftenden Filme ergeben. Durch Reaktion der Alkoxymethylgruppen der Melaminharze mit den reaktiven Stellen des Partnerharzes wird jedoch eine dreidimensionale Vernetzung erreicht, so daß Lackfilme mit hohen Gebrauchseigenschaften resultieren.

Als größter Nachteil der Melamin-Formaldehyd-Harze ist die Abspaltung von Formaldehyd bei der Applikation von Melamin-Formaldehyd-Harz-haltigen Lacken sowie beim Einbrennen dieser Lackfilme anzusehen. Es hat daher nicht an Versuchen gefehlt, formaldehydfreie Vernetzer für Lackfilme zu entwickeln. In der Praxis werden Vernetzer auf Basis von mehrfachfunktionellen Epoxiden oder auf Basis von Polyisocyanaten eingesetzt. Epoxydharze werden in 2-Komponenten-Reaktionslacken zusammen mit Polyaminen, Aminaddukten aus Epoxiden und Polyaminen, Polyamidoaminen oder Ketiminen als Härter verwendet (H. Gempeler, W. Marquardt, Kunststoffhandbuch Bd. 10 Duroplaste, Hanser Verlag München 1988, S. 1004-1018). Polyisocyanate werden in 1- oder 2-Komponenten Systemen als Vernetzer eingesetzt (G. Mennichen, W. Wieczorrek, Kunststoffhandbuch Bd. 7 Duroplaste, Hanser Verlag München 1983, S. 540-561).

In neuerer Zeit wurde ein Vernetzerprinzip veröffentlicht (z.B. EP-A 0 215 245, EP-A 0 211 432, R.K. Pinschmidt, Jr., W.F. Burgogne, D.D. Dixan and J.E. Goldstein, ACS Symp.Ser. (1988) 367, 453-466), das auf N-substituierten ω-Dialkoxyalkylaminen der allgemeinen Formel I

Z-NH-(CH₂)ₙ-CR¹(OR²)(OR³) (I)

worin
- R¹: Wasserstoff oder Alkyl,
- R² und R³: Alkyl,
- Z: einen elektronenziehenden Rest und
- n: eine ganze Zahl von 1 bis 5
bedeutet, beruht.

Die bei der sauer katalysierten Selbst- und Fremdvernetzung dieser Verbindungen ablaufenden Reaktionen sind in ACS Symp.Ser. (1988), 367, 453-466 beschrieben. Sie können sowohl mit sich selbst unter Vernetzung, als auch mit Hydroxyl- oder Aminogruppen-haltigen Polymeren oder Kondensaten unter Ausbildung kovalenter Bindungen reagieren. Ihrer Reaktion mit 1,2- bzw. 1,3-Diolgruppen-haltigen Molekülen bzw. Polymeren wie z.B. Cellulose oder Polyvinylalkohol unter Ausbildung cyclischer Acetale kommt eine besondere Bedeutung zu.

Die Verbindungen der allgemeinen Formel I können sowohl als Monomere, als auch als Polymere vorliegen. Gemäß EP-A 0 215 245 können Verbindungen der allgemeinen Formel I mit Z = Acryloyl oder Methacryloyl zu Homo- bzw. mit anderen vinylgruppenhaltigen Monomeren zu Copolymeren umgesetzt werden. ω-Dialkoxyalkylaminogruppen können auch durch polymeranaloge Umsetzung von ω-Dialkoxyalkylaminen der allgemeinen Formel II

H₂N-(CH₂)ₙ-CR¹(OR²)(OR³) (II)

worin R¹, R², R³ und n wie oben angegeben definiert sind, mit cyclische Anhydridgruppen enthaltenden Polymeren zu über Carbonsäureamid verknüpften Additionsprodukten umgesetzt werden (US-A 5,298,567). Des weiteren ist in der US-A 5,336,807 die Umsetzung von 4,4-Dimethoxybutylamin mit Isophthalsäuredimethylester zu einem monomeren Vernetzer beschrieben. In der EP-A 0 215 245 sowie der EP-A 0 211 432 sind Ammelinderivate der allgemeinen Formel III 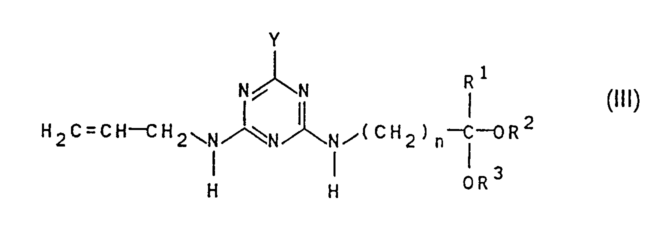 worin Y für Hydroxy steht und R¹, R², R³ und n wie oben angegeben definiert sind, beschrieben. Entsprechende Melaminderivate, in denen Y für 4,4-Diethoxybutylamino steht, sind dort ebenfalls beschrieben, konnten aber nur in einer Ausbeute von 1,5 % erhalten werden.

Es wurde nun überraschenderweise gefunden, daß in den Verbindungen der allgemeinen Formel III die Allylaminogruppe nicht erforderlich ist.

Die vorliegende Erfindung betrifft Verbindungen der allgemeinen Formel IV 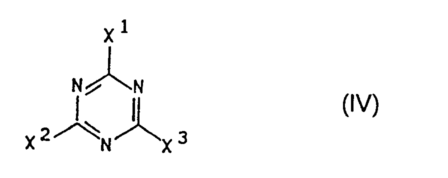 worin
X¹, X² und X³ unabhängig voneinander für Amino, (C₁-C₆)-Alkylamino, Di-(C₁-C₆)-alkylamino, Hydroxy-(C₁-C₆)-alkylamino, Di-hydroxy-(C₁-C₆)-alkylamino, (C₆-C₁₀)-Arylamino, eine Gruppe der allgemeinen Formel V 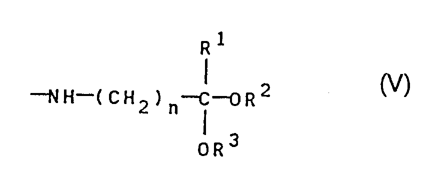 eine Gruppe der allgemeinen Formel VI 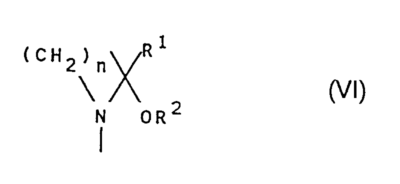 oder eine Gruppe der allgemeinen Formel VII 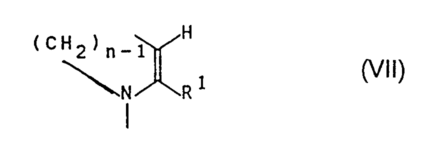 stehen;
R¹ Wasserstoff oder (C₁-C₆)-Alkyl;
R² und R³ unabhängig voneinander (C₁-C₆)-Alkyl; und
n eine ganze Zahl von 1 bis 5
bedeuten, wobei X¹, X² und X³ nicht gleichzeitig für Amino stehen können.

(C₁-C₆)-Alkyl kann geradkettig oder verzweigt sein und beispielsweise Methyl, Ethyl, n-Propyl, i-Propyl, n-Butyl, i-Butyl, sek-Butyl, tert-Butyl, n-Pentyl oder n-Hexyl bedeuten. Analoges gilt für Hydroxy-(C₁-C₆)-alkyl. (C₆-C₁₀)-Arylamino ist bevorzugt Phenylamino.

R¹ bedeutet bevorzugt Wasserstoff, während R² und R³ bevorzugt für Methyl und besonders bevorzugt für Ethyl stehen. n bedeutet bevorzugt 3.

X¹, X² und X³ stehen bevorzugt unabhängig voneinander für Amino, (C₁-C₆)-Alkylamino, Di-(C₁-C₆)-alkylamino, Hydroxy-(C₁-C₆)-alkylamino oder eine Gruppe der allgemeinen Formel V.

Besonders bevorzugt stehen X¹, X² und X³ für Amino, Butylamino, Hydroxyethylamino oder eine Gruppe der allgemeinen Formel V.

Die Verbindungen der allgemeinen Formel IV können beispielsweise in an sich bekannter Weise dadurch erhalten werden, daß man Cyanurchlorid mit Aminen der allgemeinen Formel NH₂R⁴ worin R⁴ Wasserstoff oder 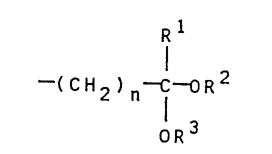 bedeutet und R¹, R², R³ und n wie obenangegeben definiert sind umsetzt und, falls einer der Reste X¹, X² oder X³ für eine Gruppe der allgemeinen Formel VI oder VII stehen soll, eine intramolekulare Halbaminalbildung und gegebenenfalls eine Eliminierung von R²OH anschließt.

Die Chloratome des Cyanurchlorids können einzeln, zu zweien oder alle drei zusammen gegen Amin substituiert werden. So ist beispielsweise bei einer Temperatur von 0 bis 5°C der Austausch nur eines Chloratoms gegen eine Amingruppe möglich, während die beiden übrigen Chloratome noch nicht reagieren. Bei einer Temperatur von 40 bis 45°C kann das zweite Chloratom ausgetauscht werden, während das dritte noch nicht reagiert (J. Am. Chem. Soc. 73 (1951) 2981-3). Schließlich kann bei Reaktionstemperaturen oberhalb von 80°C auch das dritte Chloratom substituiert werden (J. Am. Chem. Soc. 73 (1951) 2984-6). Die Verbindungen der allgemeinen Formel IV können somit durch geeignete Kombination von Temperatur und Aminen der Formel NH₂R⁴ erhalten werden.

Sollen die Reste X¹, X² und X³ verschiedene Bedeutungen haben, werden also die Chloratome des Cyanurchlorid nacheinander, d.h. schrittweise, substituiert. Sollen dagegen die Reste X¹, X² und X³ gleich sein, kann auch ein Austausch in einem Schritt erfolgen. Werden die Chloratome nacheinander ausgetauscht, so können die dabei entstehenden Zwischenverbindungen isoliert oder aber ohne Isolierung direkt weiter umgesetzt werden. Die Reihenfolge der Substitution der Chloratome ist beliebig. Sollen allerdings ein oder zwei der Reste X¹, X² und X³ für Amino stehen, so ist es bevorzugt, Cyanurchlorid zunächst mit Ammoniak umzusetzen, um dann das entstandene 2-Amino-4,6-dichlor [1,3,5]triazin bzw. 2,4-Diamino-6-chlor[1,3,5]triazin mit einem Amin 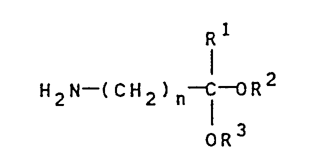 zur Reaktion zu bringen.

Als Lösungsmittel kommen für die genannten Reaktionen neben Wasser auch organische Lösungsmittel wie Ether, aliphatische oder aromatische Kohlenwasserstoffe und Ketone in Frage.

Der durch den Austausch der Chloratome am Triazinring freiwerdende Chlorwasserstoff wird vorteilhafterweise durch den Zusatz basisch reagierender Mittel gebunden. Geeignete basisch reagierende Mittel sind beispielsweise Natrium- oder Kaliumhydroxid, -carbonat oder -hydrogencarbonat oder tert.Amine, es kann aber auch ein Überschuß an Amin der Formel NH₂R⁴ eingesetzt werden. Allerdings sollte vorteilhafterweise darauf geachtet werden, daß der pH-Wert nicht zu hoch eingestellt wird, da sonst zunehmend unerwünschte Nebenprodukte, wie Cyanursäure, Ammelid- bzw. Ammelinderivate gebildet werden. Bevorzugt liegt der pH-Wert zwischen 8 und 9. Erfolgt die Umsetzung in wäßrigem Medium, kann der Zusatz geeigneter Emulgatoren von Vorteil sein. Geeignete Emulgatoren sind beispielsweise handelsübliche anionenaktive Dispergiermittel auf Basis sulfonierter Ölsäurederivate.

Sollen Verbindungen der allgemeinen Formel IV hergestellt werden, in denen ein oder mehrere Reste X¹, X² oder X³ für eine Gruppe der allgemeinen Formel VI oder VII stehen, wird an die oben beschriebene Synthese eine intramolekulare Halbaminalbildung angeschlossen. Diese Ringschlußreaktion erfolgt bevorzugt in verdünnter Lösung in Gegenwart sauer reagierender Stoffe, wie beispielsweise Essigsäure, Schwefelsäure oder p-Toluolsulfonsäure. Bei längeren Reaktionszeiten und/oder bei höheren Temperaturen schließt sich eine Eliminierung von Alkohol an, so daß auf diese Weise Verbindungen der allgemeinen Formel IV, in denen einer oder mehrere Reste X¹, X² oder X³ für eine Gruppe der allgemeinen Formel VII stehen, zugänglich sind.

Die wie oben beschrieben hergestellten erfindungsgemäßen Verbindungen der allgemeinen Formel IV können in an sich bekannter Weise isoliert werden. Beispielsweise werden sie durch Extraktion mit organischen Lösungsmitteln wie Ethern oder Carbonsäureestern, beispielsweise Diethylether oder Essigsäureethylester, von der wäßrigen Phase abgetrennt und nach Trocknung der organischen Phase mit z.B. Natrium- oder Magnesiumsulfat durch Abdestillieren des organischen Lösungsmittels isoliert.

Die erfindungsgemäßen Triazin-Verbindungen der allgemeinen Formel IV lassen sich mit konventionellen oder wasserlöslichen bzw. selbstemulgierenden hydroxylgruppen-und/oder aminogruppenhaltigen fremdvernetzenden Bindemitteln, wie Alkyd-, Polyester- oder Acrylharzen und auch mit aliphatischen Aminen und Epoxidharzen kombinieren und zu beständigen und harten Filmen aushärten.

Bei Gewichtsverhältnissen Bindemittel : erfindungsgemäße Verbindung der allgemeinen Formel IV von 30:70 bis 90:10 ist dabei die Aushärtung bei Temperaturen von 100 bis 180°C in ca. 20 Minuten erfolgt. Die Aushärtung kann unkatalysiert oder katalysiert erfolgen, wobei geeignete Katalysatoren Säuren, wie beispielsweise p-Toluolsulfonsäure, sind.

Die erfindungsgemäßen Triazin-Verbindungen der allgemeinen Formel IV können deshalb in hervorragender Weise als Härter in Lacksystemen auf Basis von hydroxylgruppenund/oder aminogruppenhaltigen Alkyd-, Polyester- oder Acrylharzen, aliphatischen Polyaminen oder Epoxidharzen und/oder deren Mischungen eingesetzt werden.

Die vorliegende Erfindung betrifft auch Lacksysteme, dadurch gekennzeichnet, daß sie eine Verbindung der allgemeinen Formel IV enthalten.

Bevorzugt betrifft die vorliegende Erfindung Lacksysteme auf Basis von hydroxylgruppen- und/oder aminogruppenhaltigen Alkyd-, Polyester- oder Acrylharzen, aliphatischen Polyaminen oder Epoxidharzen und/oder deren Mischungen, dadurch gekennzeichnet, daß sie eine erfindungsgemäße Triazin-Verbindung der allgemeinen Formel IV enthalten.

Die erfindungsgemäßen Lacksysteme können wäßrig oder nicht wäßrig sein. Beispiele für erfindungsgemäße Lacksysteme sind solche für Haushaltsgeräte beispielsweise Waschmaschinen, Kühlschränke oder Herde, insbesondere aber für den Automobilsektor. Erfindungsgemäße Lacksysteme für den Automobilsektor sind insbesondere Einbrennfüller, Basislacke, Kiarlacke und Decklacke.

Die erfindungsgemäßen Lacksysteme weisen bevorzugt ein Gewichtsverhältnis Alkyd-, Polyester- oder Acrylharz, Polyamin oder Epoxidharz: erfindungsgemäßer Verbindung der allgemeinen Formel IV von 30:70 bis 90:10 auf. Sie können im Falle von Füller-, Basis- oder Decklacksystemen im Einbrennbereich von 100 bis 200°C und einer Einbrennzeit von 10 bis 40 Minuten beziehungsweise im Falle von Basislacksystemen im forcierten Trocknungsbereich von 5 bis 20 Minuten bei 150 bis 190°C verwendet werden.

Die erfindungsgemaßen Lacksysteme können Hilfs- und Zusatzmittel enthalten, insbesondere die üblichen Lackadditive. Solche Lackadditive sind beispielsweise anorganische oder organische (Farb-)Pigmente, Füllstoffe, Antiabsetzmittel, Entschäumungs- und/oder Netzmittel, Verlaufmittel, Reaktiv-Verdünner, Weichmacher, (UV-)Stabilisatoren, Katalysatoren, Rheologiehilfsmittel, wie z.B. Mikrogele oder Polyharnstoffderivate oder zusätzliche Härter. Gegebenenfalls können die genannten Additive den erfindungsgemäßen Lacksystemen auch erst unmittelbar vor der Verarbeitung zugegeben werden.

### Beispiel 1

In einem 1-l-Dreihalskolben mit Rückflußkühler, KPG-Rührer, Innenthermometer und pH-Elektrode werden 500 g Wasser, 39,5 g (245 mmol) 4,4-Diethoxybutylamin und 35,6 g (245 mmol) 2,4-Diamino-6-chlor[1,3,5]triazin (J. Am. Chem. Soc. 73 (1951) 2983) vorgelegt und innerhalb einer Stunde auf Rückfluß (100°C) erhitzt und 10 min bei Rückfluß gerührt. Der pH-Wert der Reaktionsmischung wird im Verlauf der Reaktion durch sukzessive Zugabe von insgesamt 13 g (123 mmol) Natriumcarbonat, gelöst in 40 g Wasser, zwischen 8,0 und 9,0 gehalten. Der Ansatz geht bis auf wenig Ungelöstes in Lösung. Dann wird auf 25°C abgekühlt und das sich als weiße Masse abscheidende Umsetzungsprodukt mit insgesamt 300 ml Essigsäureethylester extrahiert. Nach Trocknen der organischen Phase mit Natriumsulfat wird das Lösungsmittel am Rotationsverdampfer abdestilliert.
Ausbeute: 53,0 g (196 mmol, 80 % d.Th.) N-(4,4-Diethoxy-butyl)melamin als hochviskoses Harz.

| Elementaranalyse: | | | |
|---|---|---|---|
| gef.: | C 48,2 %; | H 8,6 %; | N 30,5 % |
| ber. für C₁₁H₂₂N₆0₂ 0,2 H₂0: | C 48,2 %; | H 8,2 %; | N 30,7 % |

- ¹H-NMR: (DMSO): 1,1 ppm, t, 6 H, -CH₃;
1,5 ppm, 4 H, m, C-CH₂-C;
3,2 ppm, q, 2 H, N-CH₂-C;
3,35-3,60 ppm, m, 4 H, O-CH₂-C;
4,5 ppm, t, 1 H, (0-)₂CH-C;
5,8-6,2 ppm, bm, 4 H, -NH₂;
6,4 ppm , t, 1 H, -NH-C.
- ¹³C-NMR: (DMSO): 15,2 ppm, -CH₃; 24,7 ppm, -CH₂-;
30,7 ppm, -CH₂-; 40,0 ppm; N-CH₂-C;
60,4 ppm, O-CH₂-C; 102,0 ppm, O-CH-0;
166,3 ppm, Triazin-C; 167,0 ppm, Triazin-C.

### Beispiel 2

In einem 1-l-Dreihalskolben mit Rückflußkühler, KPG-Rührer, Innenthermometer und pH-Elektrode werden 600 g Wasser vorgelegt und auf 5°C abgekühlt. Dazu werden 48,4 g (300 mmol) 4,4-Diethoxybutylamin und 49,5 g (300 mmol) 2-Amino-4,6-dichlor[1,3,5]triazin (J. Am. Chem. Soc. 73 (1951) 2983) zugesetzt und der Ansatz innerhalb 30 min auf 45°C erhitzt. Der pH-Wert der Reaktionsmischung wird im Verlauf der Reaktion durch sukzessive Zugabe von insgesamt 15,9 g (150 mmol) Natriumcarbonat, gelöst in 50 g Wasser, zwischen 8,0 und 8,5 gehalten. Sobald die für den Austausch des ersten Chloratomes am Triazinring für die Neutralisation des dabei freiwerdenden Chlorwasserstoffs berechnete Menge an Natriumcarbonat verbraucht ist, werden nochmals 48,4 g (300 mmol) 4,4-Diethoxybutylamin zugegeben und der Ansatz innerhalb einer Stunde auf 90°C erhitzt. Der pH-Wert der Reaktionsmischung wird im Verlauf der Reaktion durch sukzessive Zugabe von insgesamt 15,9 g (150 mmol) Natriumcarbonat, gelöst in 50 g Wasser zwischen 8,0 und 8,5 gehalten. Sobald die berechnete Menge an Natriumcarbonat durch die Reaktion verbraucht ist, wird noch 30 min bei Rückfluß (100°C) gerührt. Die erhaltene Emulsion wird auf 25°C abgekühlt und 5 mal mit jeweils 200 ml Essigsäureethylester extrahiert. Die vereinigten organischen Phasen werden dann über Natriumsulfat getrocknet und das Lösungsmittel am Rotationsverdampfer abdestilliert.
Ausbeute: 98 g (236 mmol, 79 % d.Th.) N,N'-Bis(4,4-diethoxybutyl)melamin als viskoses Harz.

| Elementaranalyse: | | | |
|---|---|---|---|
| gef.: | C 54,7 %; | H 9,6 %; | N 20,3 % |
| ber. für C₁₉H₃₈N₆0₄: | C 55,0 %; | H 9,2 %; | N 20,3 % |

- ¹H-NMR: (DMSO): 1,1 ppm, t, 12 H, -CH₃;
1,5 ppm, 8 H, m, C-CH₂-C;
3,2 ppm, m, 4 H, N-CH₂-C;
3,35-3,60 ppm, m, 8 H, O-CH₂-C;
4,4 ppm, t, 2 H, (0-)₂CH-C;
5,75-6,15 ppm, 2 H, -NH₂;
6,25-6,60 ppm; 2 H, -NH-C;
- ¹³C-NMR: (DMSO): 15,2 ppm, -CH₃; 24,8 ppm, -CH₂;
30,8 ppm, -CH₂; 39,5 ppm, N-CH₂-C;
60,4 ppm, O-CH₂-C; 102,1 ppm, O-CH-0;
166,0 ppm, Triazin-C; 167,0 ppm, Triazin-C.

### Beispiel 3

In einem 1-l-Dreihalskolben mit Rückflußkühler, KPG-Rührer, Innenthermometer und pH-Elektrode werden 27,7 g (150 mmol) Cyanurchlorid und 0,7 g eines handelsüblichen Emulgators auf Basis eines sulfonierten Ölsäureamids in 300 g Eiswasser suspendiert. Dazu werden 24,2 g (150 mmol) 4,4-Diethoxybutylamin zugesetzt und der pH-Wert der Reaktionsmischung im Verlauf der Reaktion durch sukzessive Zugabe von insgesamt 7,95 g (75 mmol) Natriumcarbonat, gelöst in 50 g Wasser, zwischen 8,0 und 8,5 gehalten. Die Reaktionstemperatur wird bei 5'C gehalten. Sobald die für den Austausch des ersten Chloratoms am Triazinring für die Neutralisation des dabei freiwerdenden Chlorwasserstoffs berechnete Menge an Natriumcarbonat verbraucht ist (nach ca. 50 min), werden nochmals 24,2 g (150 mmol) 4,4-Diethoxybutylamin zugegeben und der Ansatz auf 40°C erwärmt. Der pH-Wert der Reaktionsmischung wird im Verlauf der Reaktion durch sukzessive Zugabe von insgesamt 7,95 g (75 mmol) Natriumcarbonat, gelöst in 50 g Wasser, zwischen 8,0 und 8,5 gehalten. Sobald die berechnete Menge an Natriumcarbonat durch die Reaktion verbraucht ist, werden nochmals 24,2 g (150 mmol) 4,4-Diethoxybutylamin zugegeben, der Ansatz auf 85°C erwärmt und der pH-Wert der Reaktionsmischung wird im Verlauf der Reaktion durch sukzessive Zugabe von insgesamt 7,95 g (75 mmol) Natriumcarbonat, gelöst in 50 g Wasser, zwischen 8,0 und 8,5 gehalten. Nachdem die berechnete Menge an Natriumcarbonat verbraucht ist, wird noch 30 min bei Rückfluß (100°C) gerührt. Die erhaltene Emulsion wird auf 25°C abgekühlt und das als schmierige Masse abgeschiedene Produkt mit Essigsäureethylester extrahiert. Die vereinigten organischen Phasen werden über Natriumsulfat getrocknet und das Lösungsmittel am Rotationsverdampfer abdestilliert.

Ausbeute: 67 g (120 mmol, 80 % d.Th.)
N,N',N''-Tris(4,4-diethoxybutyl)melamin als wachsartigen Feststoff

| Elementaranalyse: | | | |
|---|---|---|---|
| gef.: | C 56,3 %; | H 9,5 %; | N 15,3 % |
| ber. für C₂₇H₅₄N₆0₆: | C 58,0 %; | H 9,7 %; | N 15,0 % |

- ¹H-NMR: (DMSO): 1,1 ppm, t, 6 H, -CH₃;
1,4-1,6 ppm, 4 H, m, C-CH₂-C;
3,4-3,6 ppm, m, 2 H, N-CH₂-C;
3,4-3,6 ppm, m, 4 H, O-CH₂-C;
4,5 ppm, b, 1 H, (0-)₂-CH-C;
6,2-6,8 ppm, m, 1 H, NH-C.
- ¹³C-NMR: (DMSO): 15,3 ppm, -CH₃; 24,9 ppm, -CH₂;
30,9 ppm, 39,6 ppm, 40,1 ppm, -CH₂;
60,4 ppm, O-CH₂-C; 102,2 ppm, O-CH-0;
165,7 ppm, Triazin-C;
N-CH₂-C unter DMSO-Signal verdeckt.

### Beispiel 4

In einem 250-ml-Dreihalskolben mit Rückflußkühler, KPG-Rührer, Innenthermometer und pH-Elektrode werden 100 g Eiswaser, 0,25 g eines anionenaktiven Dispergiermittels auf Basis eines sulfonierten N,N-Dibutylölsäureamids sowie 10,12 g (55 mmol) Cyanurchlorid vorgelegt und 10 min bei 2°C gerührt. Dazu werden 3,37 g (55 mmol) Ethanolamin zugesetzt, der pH-Wert der Reaktionsmischung wird durch sukzessive Zugabe von insgesamt 2,9 g (27 mmol) Natriumcarbonat, gelöst in 12 g Wasser zwischen 8,0 und 8,5; die Reaktionstemperatur bei 5°C gehalten. Sobald das Natriumcarbonat durch den freiwerdenden Chlorwasserstoff verbraucht ist (nach ca. 30 min.), werden 8,90 g (55 mmol) 4,4-Diethoxybutylamin zugesetzt, der Ansatz auf 40°C aufgeheizt und der pH-Wert der Mischung durch Zugabe von insgesamt 2,9 g (27 mmol) Natriumcarbonat, gelöst in 12 g Wasser zwischen 8,0 und 8,5 gehalten. Nachdem das Natriumcarbonat verbraucht ist (ca. 30 min) werden erneut 8,90 g (55 mmol) 4,4-Diethoxybutylamin zugesetzt, der Ansatz auf 70°C aufgeheizt und der pH-Wert durch sukzessive Zugabe von insgesamt 2,9 g (27 mmol) Natriumcarbonat, gelöst in 12 g Wasser zwischen 8,0 und 8,5 gehalten. Nach Zugabe des Natriumcarbonats (ca. 15 min) wird noch 30 min bei Rückfluß (100°C) gerührt, abgekühlt und viermal mit insgesamt 400 ml Essigsäureethylester extrahiert, die organische Phase Über Na₂S0₄ getrocknet und das Lösungsmittel im Vakuum abdestilliert.
Ausbeute: 20,9 g (45,6 mmol; 83 % d.Th.) N,N'-Bis(4,4diethoxybutyl)-N''-(2-hydroxyethyl)melamin als gelbliches Wachs.

| Elementaranalyse: | | | |
|---|---|---|---|
| gef.: | C 54,0 %; | H 9,3 %; | N 18,0 % |
| ber.: | C 55,0 %; | H 9,2 %; | N 18,3 % |

- ¹H-NMR: (DMSO): 1,1 ppm, 12 H, t, -CH₃;
1,45-1,55 ppm, 8 H, m, C-CH₂-C;
3,15-3,25 ppm, 4 H, m, N-CH₂-C;
3,20-3,35 ppm, 3 H, m, N-CH₂-C;, OH;
3,35-3,60 ppm, 10 H, m, O-CH₂-C;
4,45 ppm, 2 H, t, (0-)₂CH-C;
6,1-6,7 ppm, NH
- ¹³C-NMR: (DMSO): 15,3 ppm, -CH₃; 24,8 ppm, CH₂;
30,8 ppm, CH₂; 42,7 ppm, N-CH₂;
59,7 ppm, CH₂-OH; 60,4 ppm, O-CH₂;
102,1 ppm, O-CH-0; 165,7 ppm Triazin C

### Beispiel 5

Das Verfahren gemäß Beispiel 4 wird wiederholt, wobei aber anstelle von 3,37 g Ethanolamin 4,02 g (55 mmol) n-Butylamin eingesetzt werden.
Ausbeute: 14,5 g (30,8 mmol; 56 % d.Th.) N-Butyl-N,N'bis-(4,4-diethoxybutyl)melamin als gelbliche Paste.

| Elementaranalyse: | | | |
|---|---|---|---|
| gef.: | C 56,1 %; | H 9,6 %; | N 17,9 % |
| ber.: | C 58,7 %; | H 9,9 %; | N 17,9 % |

- ¹H-NMR: (DMSO): 0,88 ppm, 3 H, t, -CH₃;
1,10 ppm, 12 H, t, CH₃;
1,20-1,35 ppm, 2 H, M, CH₂;
1,4-1,6 ppm, 10 H, m, CH₂;
3,1-3,3 ppm, 6 H, m, N-CH₂;
3,35-3,60 ppm, 8 H, m, O-CH₂;
4,45 ppm, 2 H; t, O-CH-0;
6,3-6,7 ppm, m, NH;
- ¹³C-NMR: (DMSO): 13,6 ppm, CH₃; 15,3 ppm, CH₃;
19,6 ppm, CH₂; 24,8 ppm, CH₂;
30,7 ppm, CH₂; 30,8 ppm, CH₂;
39,8 ppm, N-CH₂; 60,4 ppm, O-CH₂;
102.1 ppm, O-CH-O; 165,3 ppm, Triazin C;
165,7 ppm, Triazin-C

### Beispiel 6

In einem 500-ml-Dreihalskolben mit Rückflußkühler, KPG-Rührer, Innenthermometer und pH-Elektrode werden 13,83 g (75 mmol) Cyanurchlorid und 0,4 g eines handelsüblichen Emulgators auf Basis eines sulfonierten Ölsäureamids in einem Gemisch aus 50 g Wasser und 75 g Eis suspendiert. Dazu tropft man 5,83 g (75 mmol) einer 21,9 %igen Ammoniaklösung und anschließend 4,0 g (38 mmol) Natriumcarbonat, gelöst in 25 g Wasser bei einer Innentemperatur von 0 - 3°C so zu, daß der pH der Reaktionsmischung bei 8 bis 8,5 gehalten wird. Dann setzt man 12,10 g (75 mmol) 4,4-Diethoxybutylamin zu, erwärmt auf 35°C und hält den pH durch Zutropfen von 4,0 g (38 mmol) Natriumcarbonat, gelöst in 25 g Wasser zwischen 8 und 8,5. Dann werden erneut 12,10 g (75 mmol) 4,4-Diethoxybutylamin zugesetzt, die Reaktionsmischung auf 80°C erhitzt und der pH-Wert durch Zutropfen von insgesamt 4,0 g (38 mmol) Natriumcarbonat, gelöst in 25 g Wasser zwischen 8 und 8,5 gehalten. Anschließend wird noch 1 h bei Rückfluß gerührt, abgekühlt und die Reaktionsmischung viermal mit insgesamt 350 ml Essigssäureethylester extrahiert. Nach Trocknen der organischen Phase mit Natriumsulfat wird das Lösungsmittel im Vakuum abdestilliert.
Ausbeute: 21,2 g (51 mmol; 68 % d.Th.) N,NI-Bis(4,4-diethoxybutyl)melamin. Nach NMR ist das erhaltene Produkt mit dem aus Beispiel 2 identisch.

### Beispiel 7

Je 30 Teile erfindungsgemäßer Verbindung gemäß Beispielen 1 bis 3 werden mit jeweils 108 Teilen eines handelsüblichen fremdvernetzenden Acrylharzes (65 %ig gelöst in Xylol : Butanol 3 : 1), z.B. ®Synthacryl SC 303 (®Synthacryl ist ein eingetragenes Warenzeichen der Hoechst AG, Frankfurt am Main, Bundesrepublik Deutschland) gemischt und mit Isobutanol auf einen Feststoffgehalt von 60 % eingestellt. Diese Lackformulierungen wurden mit den in Tabelle 1 angegebenen Mengen an p-Toluolsulfonsäure (TSS) versetzt, mit Hilfe eines Aufziehdreiecks als 150 µm dicker Film auf Glasplatten appliziert und in der in Tabelle 1 angegebenen Zeit mit der in Tabelle 1 angegebenen Temperatur eingebrannt. Die erhaltenen Lacke wurden der im folgenden beschriebenen lacktechnischen Ausprüfung unterzogen:
1. Klebneigung:
   Manuelle Prüfung der Klebneigung des eingebrannten Lackfilms nach einer Abkühlung von 30 min.
2. Haftung zum Glasträger:
   Mit einer Messerklinge wird versucht, den eingebrannten Lackfilm nach Erkalten auf Zimmertemperatur vom Glasträger abzuheben.
3. Nagelhärte:
   Die Nagelhärte des eingebrannten Films wird durch Ritzen mit dem Fingernagel geprüft.
   Beurteilungsstufen: 0 = nicht nagelhart, 1 = nagelhart.
4. Pendelhärte:
   Die Pendelhärte wird mit dem Pendelgerät nach König (DIN 53157) unter Normklima geprüft.
5. Xylolfestigkeit:
   Auf die eingebrannten Lackfilme werden nach Lagerung unter Normklima (23 ^{o}C, rel. Luftfeuchtigkeit 50%) mit Xylol getränkte Wattebäusche unter einer Glaskalotte aufgesetzt und in Zeitintervallen von 5, 10 bzw. 15 min zur Seite gerückt. Die mit Xylol belastete Lackschicht wird auf Oberflächenstörungen, wie z.B. Blasen, Mattierung untersucht. Die Nagelhärte der Lackschicht wird durch Kratzen mit dem Fingernagel geprüft.
   Beurteilungsstufen in den Tabellen: 1 = nagelhart, 2 = schwer abkratzbar, 3 = abkratzbar, 4 = leicht abkratzbar, 5 = löst sich auf.
   Es wurden die in Tabelle 1 genannten Ergebnisse erhalten:

**Tabelle 1**

| Parameter | | Einbrennbedingungen | | | | |
|---|---|---|---|---|---|---|
| TSS fest/fest | | 1,5 % | 1,5 % | 1,5 % | 1,5 % | 0,0 % |
| Temp. [c'C] | | 130 | 150 | 160 | 180 | 160 |
| Zeit [min] | | 30 | 30 | 30 | 30 | 30 |

| Vernetzer | Parameter | Prüfergebnisse | | | | |
|---|---|---|---|---|---|---|
| Beispiel 1 | Klebneigung | klebt nicht | klebt nicht | klebt nicht | klebt nicht | klebt nicht |
| | Haftung auf Glasträger | gut | gut | gut | gut | gut |
| | Nagelhärte | 0 | 1 | 1 | 0 | 0 |
| | Pendelhärte | 54 | 97 | 102 | 99 | 75 |

| | Xylolfestigkeit | | | | | |
|---|---|---|---|---|---|---|
| | 5 min | | 4 | 4 | | |
| | 10 min | | 4 | 4 | | |
| | 15 min | | 4 | 5 | | |
| Beispiel 2 | Klebneigung | gering | klebt nicht | klebt nicht | klebt nicht | klebt nicht |
| | Haftung auf Glasträger | gut | gut | gut | gut | gut |
| | Nagelhärte | 0 | 1 | 1 | 1 | 0 |
| | Pendelhärte | 23 | 112 | 112 | 112 | 21 |

| | Xylolfestigkeit | | | | | |
|---|---|---|---|---|---|---|
| | 5 min | 4 | 3 | 1 | 1 | |
| | 1 0 min | 4 | 3 | 2 | 1 | |
| | 15 min | 4 | 3 | 2 | 2 | |
| Beispiel 3 | Klebneigung | klebt nicht | klebt nicht | klebt nicht | klebt nicht | klebt nicht |
| | Haftung auf Glasträger | gut | gut | gut | gut | gut |
| | Nagelhärte | 0 | 0 | 1 | 0 | 1 |
| | Pendelhärte | 43 | 108 | 109 | 107 | 123 |

| | Xylolfestigkeit | | | | | |
|---|---|---|---|---|---|---|
| | 5 min | 4 | 2 | 2 | 3 | 3 |
| | 10 min | 4 | 2 | 2 | 3 | 3 |
| | 15 min | 4 | 2 | 2 | 3 | 3 |

### Beispiel 8:

Je 30 Teile erfindungsgemäße Verbindung gemäß Beispiele 1 bis 3 wurden mit jeweils 93 Teilen eines handelsüblichen fremdvernetzenden Acrylharzes (75%ig gelöst in ®Solvesso 100; ®Solvesso ist ein eingetragenes Warenzeichen der Firma Exxon, USA), z.B. (®Synthacryl SC 370, gemischt und mit Isobutanol auf einen Feststoffgehalt von 60 % eingestellt. Diese Lackformulierungen wurden mit den in Tabelle 2 angegebenen Mengen an p-Toluolsulfonsäure (TSS) versetzt, als 150 µm dicker Film auf Glasplatten aufgerakelt, in der in Tabelle 2 angegebenen Zeit mit der in Tabelle 2 angegebenen Temperatur eingebrannt und, wie in Beispiel 7 angegeben, lacktechnisch ausgeprüft. Es wurden die in Tabelle 2 genannten Ergebnisse erhalten.

**Tabelle 2**

| Parameter | | Einbrennbedingungen | | |
|---|---|---|---|---|
| TSS fest/fest | | 1,5 % | 15% | 2,0 % |
| Temp. [OC] | | 160 | 180 | 180 |
| Zeit [min] | | 30 | 30 | 30 |

| Vernetzer | Parameter | Prüfergebnisse | | |
|---|---|---|---|---|
| Beispiel 1 | Klebneigung | klebt nicht | klebt nicht | klebt nicht |
| | Haftung auf Glasträger | gut | gut | gut |
| | Nagelhärte | | | |
| | Pendelhärte | 14 | 8 | 10 |

| | Xylolfestigkeit | | | |
|---|---|---|---|---|
| | 5 min | | | |
| | 10 min | | | |
| | 15 min | | | |
| Beispiel 2 | Klebneigung | klebt nicht | klebt nicht | klebt nicht |
| | Haftung auf | | | |
| | Glasträger | gut | gut | gut |
| | Pendelhärte | 55 | 40 | 34 |

| Vernetzer | Parameter | Prüfergebnisse | | |
|---|---|---|---|---|
| | Xylolfestigkeit | | | |
| | 5 min | | | |
| | 10 min | | | |
| | 15 min | | | |
| Beispiel 3 | Klebneigung | klebt nicht | klebt nicht | klebt nicht |
| | Haftung auf Glasträger | gut | gut | gut |
| | Pendelhärte | 38 | 36 | 28 |

| | Xylolfestigkeit | | | |
|---|---|---|---|---|
| | 5 min | | 3 | |
| | 10 min | | 4 | |
| | 15 min | | 4 | |

### Beispiel 9

Je 30 Teile erfindungsgemäße Verbindung gemäß Beispiele 1 bis 3 wurden mit jeweils 117 Teilen eines handelsüblichen ölfreien Polyesterharzes (60%ig gelöst in Xylol), z.B.®Alftalat AR 351 (®Alftalat ist ein eingetragenes Warenzeichen der Hoechst AG, Frankfurt am Main, Bundesrepublik Deutschland) gemischt und mit Isobutanol auf einen Feststoffgehalt von 60 % eingestellt. Diese Lackformulierungen wurden mit den in Tabelle 3 angegebenen Mengen an p-Toluolsulfonsäure (TSS) versetzt, als 150 µm dicker Film auf Glasplatten aufgerakelt, in der in Tabelle 3 angegebenen Zeit mit der in Tabelle 3 angegebenen Temperatur eingebrannt und, wie in Beispiel 7 angegeben, lacktechnisch ausgeprüft. Es wurden die in Tabelle 3 genannten Ergebnisse erhalten.

**Tabelle 3**

| Parameter | | Einbrennbedingungen | | | |
|---|---|---|---|---|---|
| TSS fest/fest | | 1,5 % | 1,5 % | 2,0 % | 1,5 % |
| Temp. [°C] | | 160 | 180 | 180 | 160 |
| Zeit [min] | | 30 | 30 | 30 | 30 |
| DEG-Zusatz | | 0 | 0 | 0 | 1% |

| Vernetzer | Paramter | Prüfergebnisse | | | |
|---|---|---|---|---|---|
| Beispiel 1 | Klebneigung | klebt nicht | klebt nicht | klebt nicht | klebt nicht |
| | Haftung auf Glasträger | gut | gut | gut | gut |
| | Nagelhärte | 0 | 0 | 1 | 1 |
| | Pendelhärte | 27 | 21 | 56 | 123 |

| | Xylolfestigkeit | | | | |
|---|---|---|---|---|---|
| | 5 min | | | | 2 |
| | 10 min | | | | 3 |
| | 15 min | | | | 3 |
| Beispiel 2 | Klebneigung | klebt nicht | klebt nicht | klebt nicht | |
| | Haftung auf Glasträger | gut | gut | gut | |
| | Nagelhärte | 1 | 1 | 1 | |
| | Pendelhärte | 87 | 73 | 76 | |

| | Xylolfestigkeit | | | | |
|---|---|---|---|---|---|
| | 5 min | | | | |
| | 10 min | | | | |
| | 15 min | | | | |
| Beispiel 3 | Klebneigung | klebt nicht | klebt nicht | klebt nicht | |
| | Haftung auf Glasträger | gut | gut | gut | |
| | Nagelhärte | 1 | 1 | 1 | |
| | Pendelhärte | 85 | 85 | 80 | |

| | Xylolfestigkeit | | | | |
|---|---|---|---|---|---|
| | 5 min | 2 | 1 | | |
| | 10 min | 3 | 2 | | |
| | 15 min | 3 | 3 | | |

### Beispiel 10

Je 30 Teile erfindungsgemäßer Verbindung gemäß Beispiele 1 bis 3 wurden mit jeweils 93 Teilen eines handelsüblichen wasserverdünnbaren Alkydharzes (75%ig gelöst in Butanol und Butylglykol), z.B. ®Resydrol WY 323 (®Resydrol ist ein eingetragenes Warenzeichen der Hoechst AG, Frankfurt am Main, Bundesrepublik Deutschland) gemischt und mit Isobutanol auf einen Feststoffgehalt von 60 % eingestellt. Diese Lackformulierungen wurden mit den in Tabelle 4 angegebenen Mengen an p-Toluolsulfonsäure (TSS) versetzt, als 150 µm dicker Film auf Glasplatten aufgerakelt, in der in Tabelle 4 angegebenen Zeit mit der in Tabelle 4 angegebenen Temperatur eingebrannt und, wie in Beispiel 7 angegeben, lacktechnisch ausgeprüft. Es wurden die in Tabelle 4 genannten Ergebnisse erhalten.

**Tabelle 4**

| | Parameter | Einbrennbedingungen | |
|---|---|---|---|
| | TSS fest/fest | 1,5 % | 1,5 % |
| | Temp. [OCl | 160 | 180 |
| | Zeit [min] | 30 | 30 |

| Vernetzer | Parameter | Prüfergebnisse | |
|---|---|---|---|
| Beispiel 1 | Klebneigung | klebt nicht | klebt nicht |
| | Haftung auf Glasträger | gut | gut |
| | Nagelhärte | 1 | 1 |
| | Pendelhärte | 131 | 131 |

| | Xylolfestigkeit | | |
|---|---|---|---|
| | 5 min | 2 | 2 |
| | 10 min | 2 | 3 |
| | 15 min | 2 | 3 |
| Beispiel 2 | Klebneigung | klebt nicht | klebt nicht |
| | Haftung auf Glasträger | gut | gut |
| | Nagelhärte | 1 | 1 |
| | Pendelhärte | 128 | 128 |

| | Xylolfestigkeit | | |
|---|---|---|---|
| | 5 min | 1 | 1 |
| | 10 min | 2 | 2 |
| | 15 min | 2 | 3 |
| Beispiel 3 | Klebneigung | klebt nicht | klebt nicht |
| | Haftung auf Glasträger | gut | gut |
| | Nagelhärte | 0 | 0 |
| | Pendelhärte | 115 | 115 |

| | Xylolfestigkeit | | |
|---|---|---|---|
| | 5 min | 3 | 3 |
| | 10 min | 3 | 3 |
| | 15 min | 3 | 3 |

### Beispiel 11

Je 30 Teile erfindungsgemäßer Verbindung gemäß Beispiele 2 bis 3 wurden mit jeweils 140 Teilen eines handelsüblichen feuchtigkeitshärtenden Einkomponenten-Polyurethan-harzes (50%ig gelöst in Xylol/Butylacetat), z.B. ®Beckocoat PU 428 (®Beckocoat ist ein eingetragenes Warenzeichen der Hoechst AG, Frankfurt am Main, Bundesrepublik Deutschland) gemischt und mit Isobutanol auf einen Feststoffgehalt von 60 % eingestellt. Diese Lackformulierungen wurden mit den in Tabelle 5 angegebenen Mengen an p-Toluolsulfonsäure (TSS) versetzt, als 150 µm dicker Film auf Glasplatten aufgerakelt, in der in Tabelle 5 angegebenen Zeit mit der in Tabelle 5 angegebenen Temperatur eingebrannt und, wie in Beispiel 7 angegeben, lacktechnisch ausgeprüft. Es wurden die in Tabelle 5 genannten Ergebnisse erhalten.

**Tabelle 5**

| | Parameter | Einbrennbedingungen | |
|---|---|---|---|
| | TSS fest/fest | 1,5 % | 1,5 % |
| | Temp. [OC] | 160 | 180 |
| | Zeit [min] | 30 | 30 |

| Vernetzer | Parameter | Prüfergebnisse | |
|---|---|---|---|
| Beispiel 2 | Klebneigung | klebt nicht | klebt nicht |
| | Haftung auf Glasträger | gut | gut |
| | Pendelhärte | 105 | 104 |

| | Xylolfestigkeit | | |
|---|---|---|---|
| | 5 min | 1 | 1 |
| | 10 min | 1 | 1 |
| | 15 min | 2 | 1 |
| Beispiel 3 | Klebneigung | klebt nicht | klebt nicht |
| | Haftung auf Glasträger | gut | gut |
| | Pendelhärte | 85 | 75 |

| | Xylolfestigkeit | | |
|---|---|---|---|
| | 5 min | 1 | 1 |
| | 10 min | 2 | 1 |
| | 15 min | 3 | 2 |

### Beispiel 12

36,5 Teile erfindungsgemäßer Verbindung gemäß Beispiel 2 wurden mit 50 Teilen eines handelsüblichen modifizierten aliphatischen Polyamins z.B. ®Beckopox EH 611 (®Beckopox ist ein eingetragenes Warenzeichen der Hoechst AG, Frankfurt am Main, Bundesrepublik Deutschland) gemischt und mit Isobutanol auf einen Feststoffgehalt von 60 % eingestellt. Diese Lackformulierung wurde mit 1,5 % p-Toluolsulfonsäure (40%ig, fest/fest) versetzt, als 150 µ dicker Film auf eine Glasplatte aufgerakelt und 30 min bei 160 ^{o}C eingebrannt. Die lacktechnische Ausprüfung gemäß Beispiel 7 ergab folgende Ergebnisse:

| | |
|---|---|
| Klebneigung: | klebt nicht |
| Haftung auf Glasträger: | gut |
| Nagelhärte: | 1 |
| Pendelhärte: | 130 |
| Xylolfestigkeit: | 5 min 1 |
| | 10 min 1 |
| | 15 min 1 |

## Patentansprüche

1. Verbindungen der allgemeinen Formel IV 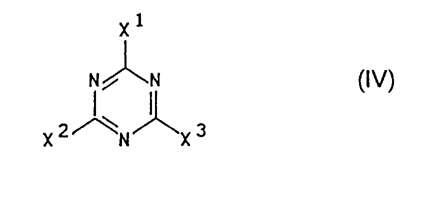 worin
X¹, X² und X³ unabhängig voneinander für Amino, (C₁-C₆)Alkylamino, Di-(C₁-C₆)-alkylamino, Hydroxy-(C₁-C₆)alkylamino, Dihydroxy-(C₁-C₆)-alkylamino, (C₆-C₁₀)-Arylamino, eine Gruppe der allgemeinen Formel V 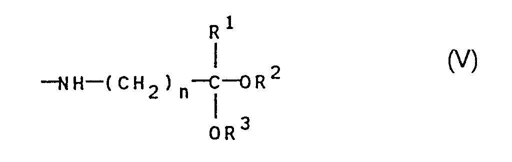 oder eine Gruppe der allgemeinen Formel VI 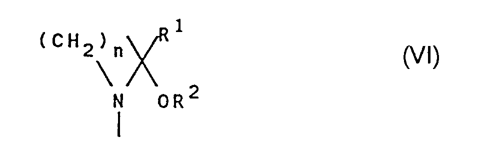 oder eine Gruppe der allgemeinen Formel VII 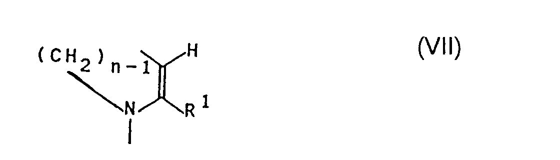 stehen;
R¹ Wasserstoff oder (C₁-C₆)-Alkyl;
R² und R³ unabhängig voneinander (C₁-C₆)-Alkyl; und
n eine ganze Zahl von 1 bis 5
bedeuten, wobei X¹, X² und X³ nicht gleichzeitig für Amino stehen können.

2. Verbindungen gemäß Anspruch 1, dadurch gekennzeichnet, daß R¹ Wasserstoff bedeutet.

3. Verbindungen gemäß Anspruch 1 und/oder 2, dadurch gekennzeichnet, daß R² und R³ für Methyl, besonders bevorzugt für Ethyl, stehen.

4. Verbindungen gemäß einem oder mehreren der Ansprüche 1 bis 3, dadurch gekennzeichnet, daß n 3 bedeutet.

5. Verbindungen gemäß einem oder mehreren der Ansprüche 1 bis 4, dadurch gekennzeichnet, daß X¹, X² und X³ unabhängig voneinander für Amino, Butylamino, Hydroxyethylamino oder eine Gruppe der allgemeinen Formel V steht.

6. Verfahren zur Herstellung der Verbindungen der allgemeinen Formel IV gemäß einem der Ansprüche 1 bis 4, dadurch gekennzeichnet, daß man Cyanurchlorid mit Aminen der allgemeinen Formel NH₂R⁴ worin R⁴ Wasserstoff oder 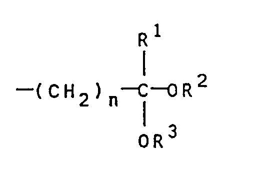 bedeutet und R¹, R², R³ und n wie obenangegeben definiert sind umsetzt und, falls einer der Reste X¹, X² oder X³ für eine Gruppe der allgemeinen Formel VI oder VII stehen soll, eine intramolekulare Halbaminalbildung und gegebenenfalls eine Eliminierung von R²OH anschließt.

7. Verwendung einer Verbindung der allgemeinen Formel IV gemäß einem der Ansprüche 1 bis 5 als Vernetzer für hydroxyl- oder aminogruppenhaltige Lackbindemittel.

8. Lacksystem, dadurch gekennzeichnet, daß es eine Verbindung der allgemeinen Formel IV gemäß einem der Ansprüche 1 bis 5 enthält.
